# EUROPEAN PATENT APPLICATION

(11) **EP 4 376 013 A1**
(43) Date of publication of application: **29.05.2024**
(21) Application number: 22845684.4
(22) Date of filing: 23.05.2022
(51) Int. Cl.: G16H 10/40, G06Q 10/06

(54) **LABORATORY OPERATION MANAGEMENT SYSTEM, OPERATION MANAGEMENT SERVER, AND OPERATION MANAGEMENT METHOD**

(30) Priority: 21.07.2021 JP 2021120447
(71) Applicant: HITACHI HIGH-TECH CORPORATION, Tokyo 105-6409 (JP)
(72) Inventor: KAWASAKI, Kenji, Tokyo 105-6409 (JP); KOIZUMI, Nobuhiro, Tokyo 105-6409 (JP); NAKAJIMA, Aika, Tokyo 105-6409 (JP); SEKI, Yoshihiro, Tokyo 105-6409 (JP)
(74) Representative: MERH-IP Matias Erny Reichl Hoffmann Patentanwälte PartG mbB
(86) International application number: PCT/JP2022/021082
(87) International publication number: WO 2023/002747

(57) **Abstract**

The invention assists with communication between laboratory technicians, and promotes cooperation between the laboratory technicians. For this purpose, the invention involves, in response to an alarm from an automated analysis device: estimating, on the basis of sensor data 203A regularly transmitted from a first mobile device 102A, a status including a location and an action of a first laboratory technician holding the first mobile device 102A that has been registered for a planned handler; estimating, on the basis of sensor data 205B regularly transmitted from a second mobile device 102B, a status including a location and an action of a second laboratory technician holding the second mobile device 102B that has been registered for a helper; and identifying a handler for resolving the alarm of the automated analysis device on the basis of the statuses that the first laboratory technician and the second laboratory technician immediately before a timing where the alarm of the automated analysis device was resolved.

## Description

### Technical Field

The present invention relates to a laboratory operation management system, an operation management server, and an operation management method assisting with communication between laboratory technicians in a laboratory in which automated analysis devices for spectroscopic analysis, emission spectrochemical analysis, chromatographic analysis, mass spectrometric analysis, or the like are used.

### Background Art

In recent years, because of difficulties of labor conditions, difficulties of relations with patients, or the like, a lack of professional hospital staffs such as doctors or nurses working in hospitals has become a critical problem. An improvement in motivation is important on both an organization side providing medical services and a patient side needing the medical services (see NPL 1).

The laboratory technicians, one of the hospital professions, are extremely busy performing operations of measuring specimens using the automated analysis devices. In the laboratory, a plurality of the laboratory technicians cooperate with each other to perform operations, but it is difficult to know who needs help in the laboratory. Further, although the laboratory technician is helped, the help is not necessarily assessed. In such an environment, it is difficult to promote cooperation and it is also difficult to improve motivation of the laboratory technicians.

As a system for the purpose of improving motivation, a communication system in an organization in PTL 1 includes: a coin presentation means that presents a specific virtual coin selected from a plurality of types of virtual coins representing appreciation, a coin acquisition means that acquires the virtual coin, a number-of-acquisition storage means that stores the number of acquisitions of the virtual coin acquired by an individual or each department, and a number-of-acquisition display means that displays the number of acquisitions of the virtual coin acquired by an individual or each department. Since appreciation can be delivered freely by structuring "Thanks" through the virtual coin, it is possible to obtain the effect of motivating people and vitalizing the organization

As a system configured to reduce a load on a laboratory technician in a laboratory facility, there is a system in PTL 2 in which communication can be made as necessary through a communication means between the laboratory facility where an analysis operation is performed using an automated analysis device or the like and a service company that receives data such as an operation status of the automated analysis device in real time via a communication line and performs service operations such as analysis, diagnosis, maintenance, and inspection of various types of data. Since the service company remotely assists with analysis preparation work or device ending work as an agency, the laboratory technician is free from these working loads.

### Citation List

### Patent Literature

PTL 1: JP2017-102891A
PTL 2: JP2004-028670A

### Non Patent Literature

NPL 1: Iwata Sachiyo, Uemura Mami, Konomi Masahiro, Nagai Yayoi, Higashiyama Katsuhiko, "Study on Motivation of Medical Profession and Motivator - Focus on Comparative Analysis between Occupations -" Shodai Business Review 2(2), 225-235, 2013-03, Graduate School of Social Sciences, Department of Professional Business, University of Hyogo

### Summary of Invention

### Technical Problem

PTL 1 discloses that user A presents a coin representing appreciation to user B. PTL 2 discloses that quick countermeasures can be made by contacting a service staff close to a laboratory facility when a trouble cannot be resolved by remote assistance. That is, in these systems, a partner is designated directly or a partner is selected according to a simple condition such as proximity, to help or deliver appreciation to the partner.

However, generally, if the laboratory technician in the laboratory does not express a strong appeal, other laboratory technicians may not know whether assistance is necessary or not necessary in many cases. Not only alarms that require high skills and experience, but also simple alarms of the automated analysis devices (for example, a remaining amount of a reagent is small) may be difficult to take countermeasures because the other laboratory technicians are busy with other tasks. In this way, it is considered that, in many situations, cooperation is not promoted even if there are laboratory technicians who can afford to help, since the laboratory technician struggling in the laboratory is not understood by the other laboratory technicians (due to a lack of visibility).

When a certain laboratory technician falsely reports that he or she performs a work for resolving a device alarm, in a system in which the false report is used as it is for assessment, there is a concern that promotion of the cooperation may be hindered by malicious laboratory technicians.

The invention has been devised in view of the foregoing circumstances and an object of the invention is to provide a system capable of improving motivation of the laboratory technician by performing list display (visibility) of a state of each automated analysis device, a planned handler of an alarm, and a status of the planned handler, and promoting the cooperation and capable of assessing the laboratory technician actually resolving a device alarm by associating a change in a device state and a status of the laboratory technician.

### Solution to Problem

According to an embodiment of the invention, a laboratory operation management system includes: a plurality of automated analysis devices that are installed in a laboratory and configured to analyze biological samples; a mobile device that is held by each laboratory technician performing an operation in the laboratory; and an operation management server that includes a data accumulation unit, a notifying unit, a status estimation unit, and a handler identifying unit.

The data accumulation unit accumulates device state data indicating a device state regularly transmitted from each of the plurality of automated analysis devices in a storage device. The notifying unit requests handling from the mobile device when the device state indicated by the device state data transmitted from a first automated analysis device represents that an alarm is being generated. The status estimation unit estimates a status including a location and an action of a first laboratory technician holding a first mobile device on the basis of sensor data regularly transmitted from the first mobile device that registers a planned handler in response to the request for the handling from the notifying unit, and accumulates the status in the storage device. The notifying unit requests help from the mobile device when the device state indicated by the device state data transmitted from the first automated analysis device still represents that the alarm is being generated and continues for a predetermined period or more even after the planned handler is registered. The status estimation unit estimates a status including a location and an action of a second laboratory technician holding a second mobile device on the basis of sensor data regularly transmitted from the second mobile device that registers a helper in response to the help from the notifying unit, and accumulates the status in the storage device. The handler identifying unit identifies a handler for resolving the alarm of the first automated analysis device on the basis of the status of the first laboratory technician and the status of the second laboratory technician immediately before a timing at which the alarm of the first automated analysis device accumulated in the storage device is resolved.

### Advantageous Effects of Invention

It is possible to assist with communication between laboratory technicians and promote cooperation between the laboratory technicians. Other problems, configurations, and effects will be apparent from description of the following embodiments.

### Brief Description of Drawings

[Fig. 1A] Fig. 1A is a diagram illustrating a configuration example of a laboratory operation management system.
[Fig. 1B] Fig. 1B is a functional block diagram illustrating an operation management server.
[Fig. 2] Fig. 2 is a diagram illustrating a processing sequence example of the laboratory operation management system.
[Fig. 3] Fig. 3 is a flowchart illustrating a processing example of a device state and handler list display terminal.
[Fig. 4] Fig. 4 is a flowchart illustrating a processing example of the operation management server.
[Fig. 5] Fig. 5 is a diagram illustrating a screen example of the device state and handler list display terminal.
[Fig. 6] Fig. 6 is a diagram illustrating a screen example of a mobile device.
[Fig. 7] Fig. 7 is a diagram illustrating a configuration example of a device state management table.
[Fig. 8] Fig. 8 is a diagram illustrating a configuration example of a user status management table.
[Fig. 9] Fig. 9 is a diagram illustrating a configuration example of a point management table.
[Fig. 10] Fig. 10 is a diagram illustrating a screen example of an operation analysis terminal.

### Description of Embodiments

Hereinafter, an embodiment of the invention will be described in detail with reference to the drawings.

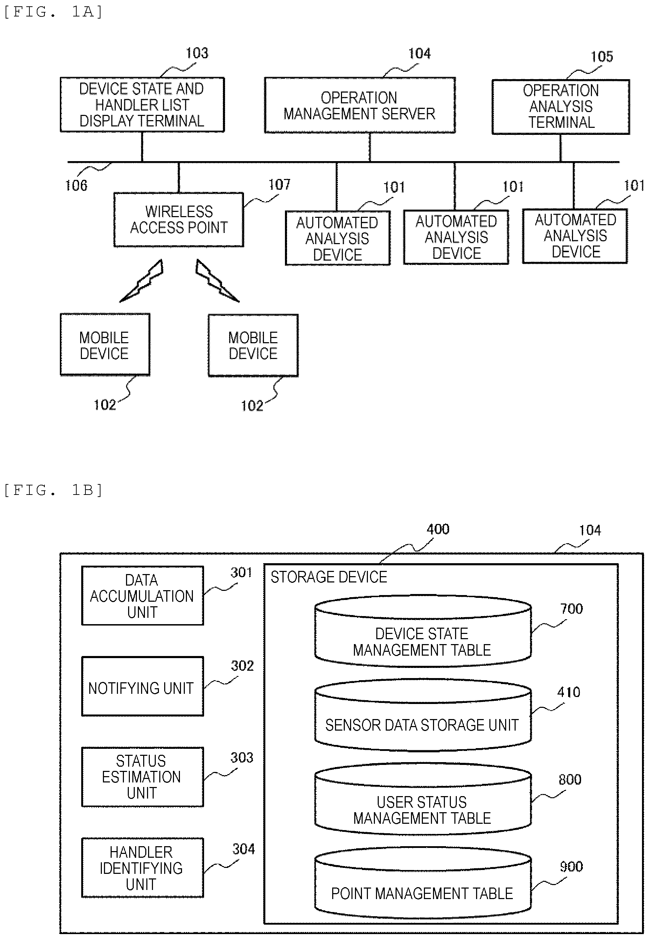

Fig. 1A is a diagram illustrating a configuration example of a laboratory operation management system according to the present embodiment. The system is constructed in units of laboratories. A plurality of automated analysis devices 101 are devices disposed in the laboratory that biochemically or immunologically analyze specimens such as blood and urine and photochemically or electrically detect reactions occurring between minute analysis target substances in the specimens and inspection reagent components. A plurality of mobile devices 102 are devices that each includes a sensor detecting a position or a status of a laboratory technician holding the mobile device and an input unit giving an instruction to register a planned handler or a helper of a work for resolving an alarm of the automated analysis device. As the sensor, a sensor for GPS, Bluetooth low energy, or acceleration can be taken into consideration. As the input unit, a button, a touch panel, a sound recognition device, a gesture recognition device, or the like can be taken into consideration. As the device, a smartphone, a tablet, a smartwatch, a headset, or the like including the sensor and the input unit can be taken into consideration.

A device state and handler list display terminal 103 is a terminal that performs list display of a device state of each automated analysis device 101, a planned handler at the time of generation of an alarm, and the like. For example, the device state and handler list display terminal 103 is disposed at a location that is easily visible to a laboratory technician in the laboratory. An operation management server 104 is a server that accumulates log data of a date and time in which a change in a state of the automated analysis device 101 occurs, a content of a device state, a planned handler at the time of generation of an alarm, a help handler, and the like, and manages an operation of the laboratory technician. Further, a point assigned to the help handler is also managed. An operation analysis terminal 105 is a device that analyzes data accumulated in the operation management server 104 and analyzes an operation situation of each laboratory technician (the number of alarm handlings, the number of helps, and the like for each period of time) or a state of the automated analysis device (for example, daily aggregation of a total time in which an alarm generates and the device is stopped during a day). These devices are connected to be able to communicate via a wired network 106 and a wireless access point 107.

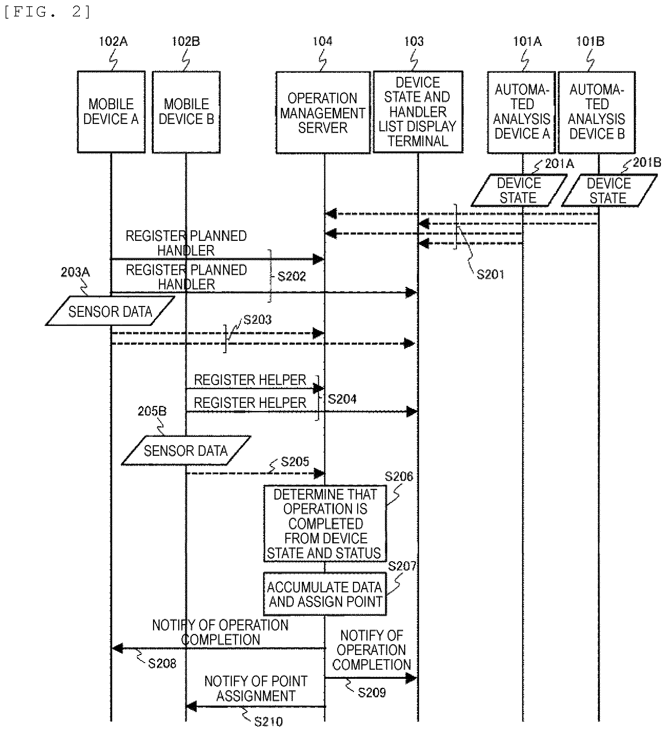

Fig. 2 is a diagram illustrating a processing sequence example of the laboratory operation management system. The automated analysis device 101 regularly transmits device state data 201 to the operation management server 104 and the device state and handler list display terminal 103 (S201). For example, at intervals of 5 seconds, the automated analysis device 101 transmits the device state data 201 representing that standby is in progress, analysis is in progress, an alarm is being generated (including alarm content), and maintenance is in progress, or stopping is in progress. In Fig. 2, communication regularly performed is indicated by a broken line. For example, it is assumed that an automated analysis device A transmits device state data 201A representing that an alarm is being generated. A laboratory technician A can ascertain that the automated analysis device A is generating an alarm with a mobile device 102A held by the laboratory technician A and register the planned handler. For example, the laboratory technician A holding the mobile device A determines to perform alarm handling of the automated analysis device A, and the laboratory technician A registers the planned handler by pressing an alarm handling button in response to the alarm of the automated analysis device A displayed as a GUI of the mobile device A or inputting a voice "The laboratory technician A performs handling of the alarm of the automated analysis device A" from a headset (S202) . The planned handler is registered in the operation management server 104 and the device state and handler list display terminal 103.

After the planned handler is registered, the mobile device 102A of the planned handler regularly transmits sensor data 203A of the planned handler to the operation management server 104 and the device state and handler list display terminal 103 (S203). The operation management server 104 and the device state and handler list display terminal 103 estimate a status of the planned handler from the received sensor data 203A. Here, for the status of the planned handler, it is assumed that the server 104 and the terminal 103 each independently estimates the status of the planned handler, but the server 104 may perform estimation and transmit an estimation result to the terminal 103. When the planned handler (the laboratory technician A) does not start the work for resolving an alarm for a given time or more, another laboratory technician (for example, a laboratory technician B) can register the helper using his or her own mobile device 102 (S204). After the helper is registered, sensor data 205B of the helper is regularly transmitted to the operation management server 104 (S205). The operation management server 104 determines who performs work to return the device state of the automated analysis device A from an abnormal state to a normal state by comparing a change in a state read from the device state data 201A of the automated analysis device A with the statuses read from the sensor data 203A of the planned handler and the sensor data 205B of the helper (S206). When the log data is accumulated and the helper (the laboratory technician B) performs alarm handling corresponding to the automated analysis device A, the number of points to be assigned to the helper is calculated and assigned (S207). Thereafter, a work completion notification is transmitted to the mobile device 102A of the planned handler (the laboratory technician A) and the device state and handler list display terminal 103 (S208 and S209). A point assignment notification is transmitted to the mobile device 102B of the helper (the laboratory technician B) (S210).

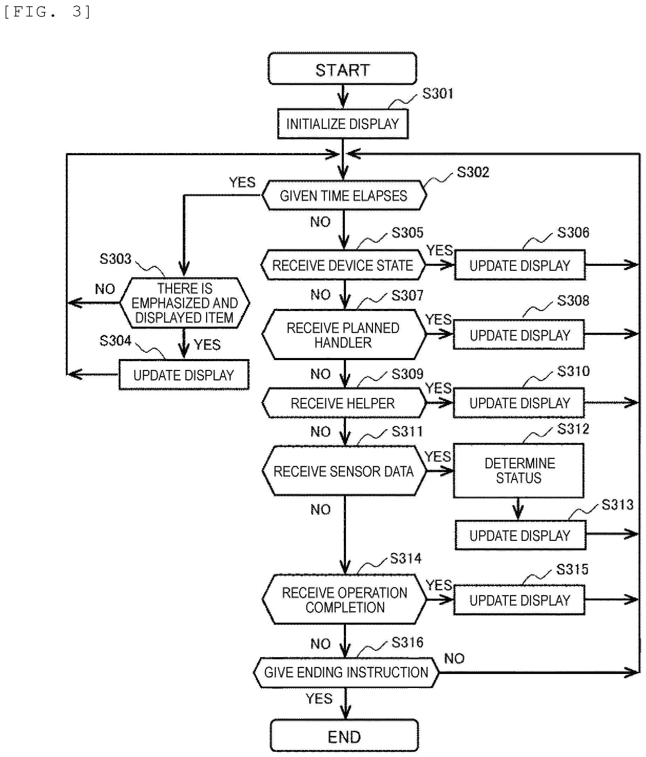

Next, a processing example of the device state and handler list display terminal 103 will be described with reference to the flowchart of Fig. 3. First, a display screen is initialized (S301).

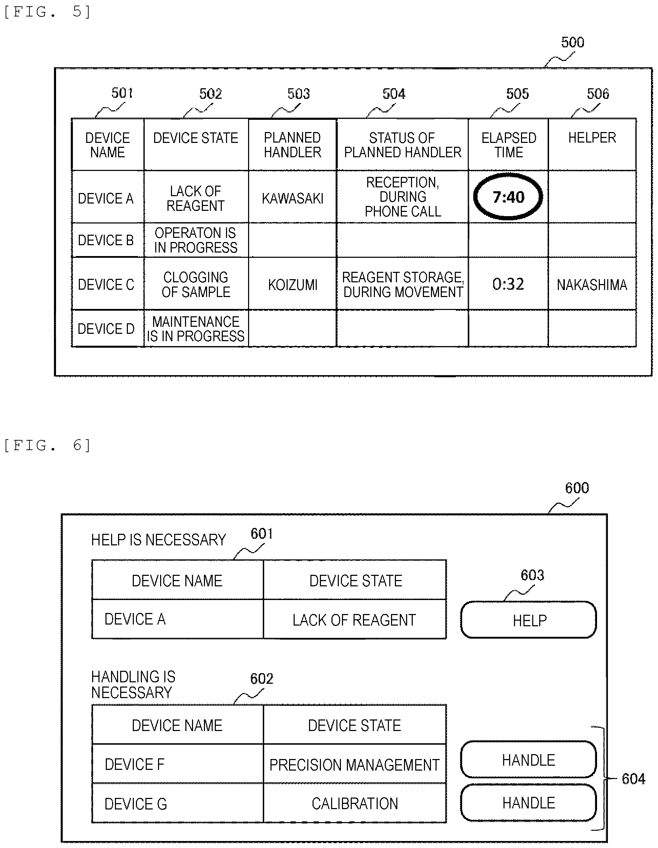

Fig. 5 illustrates a display example of the device state and handler list display terminal 103. A device name 501, a device state 502, a planned handler 503, a status 504 of the planned handler, an elapsed time 505, and a helper 506 are displayed on a screen 500 for each automated analysis device 101. The elapsed time 505 is an elapsed time after the device state data 201 representing that an alarm is being generated is first received from the device.

Subsequently, it is inspected whether the given time elapses from the previous check (for example, every 5 seconds) (S302). When the elapsed time does not elapse, the following processing is performed. When the device state data 201 is received from the automated analysis device 101 (S305), the device state 502 of the corresponding device is updated (S306). When an instruction to register the planned handler is received from the mobile device 102 (S307), the planned handler 503 of the corresponding device is updated (S308).

Fig. 6 illustrates a screen example of the mobile device 102. A list 601 of a device name and a device state in which help is necessary is displayed on a screen 600. Here, a device in which the help is necessary is a device in which the planned handler does not start the work for resolving an alarm for the given time or more. A person holding the mobile device 102 can be registered as a helper for a corresponding device by displaying a help button 603 for each device and pressing the help button. Similarly, a list 602 of a device name and a device state in which alarm handling is necessary is displayed on the screen 600. The person holding the mobile device 102 can be registered as a planned handler of a corresponding device by displaying a handling button 604 for each device and pressing the handling button. The operation management server 104 controls a handling request for the mobile device 102 or help request display.

When an instruction to register the helper is received from the mobile device 102 (S309), the helper 506 of the corresponding device is updated (S310). When the sensor data 203 is received from the mobile device 102 of the planned handler (S311), the status of the planned handler is determined from the sensor data 203 (S312) and the status 504 of the planned handler of the corresponding device is updated (S313). For example, when the sensor data 203 is GPS coordinate data, a location where there is the planned handler in a hospital or the laboratory can be determined from the coordinates. When the sensor data is beacon reception data such as Bluetooth low energy, a location where there is the planned handler can be determined from a beacon ID. When the sensor data is data of an acceleration sensor, it can be determined from the acceleration whether it is walking, stopping, etc. When the sensor data is data of an acceleration sensor attached to an arm, for example, work contents such as reagent exchange or nozzle adjustment can be determined from acceleration.

When the work completion notification is received from the operation management server 104 (S314), the device state 502 of the corresponding device is updated (S315). Further, the planned handler 503, the status 504 of the planned handler, the elapsed time 505, and the helper 506 of the corresponding device are updated to blanks. When a system manager inputs an ending instruction command (S316), the processing program ends.

When the given time elapses from the previous check in step S302, it is inspected whether there is an item emphasized and displayed on the screen 500 (Fig. 5) (S303). When there is the corresponding item, the corresponding item of the corresponding device is emphasized and displayed to draw an attention of the laboratory technician or a manager of a laboratory technician operation (S304). For example, when the planned handler is registered and then the given time or more elapses, and the device state still represents that an alarm is being generated or when the alarm is generated and then the given time or more elapses, and the planned handler is not registered, the elapsed time 505 is emphasized and displayed. As a method for the emphasis and display, it is considered that, for example, a corresponding row is displayed with noticeable color, blanking display is performed, or display text is enlarged.

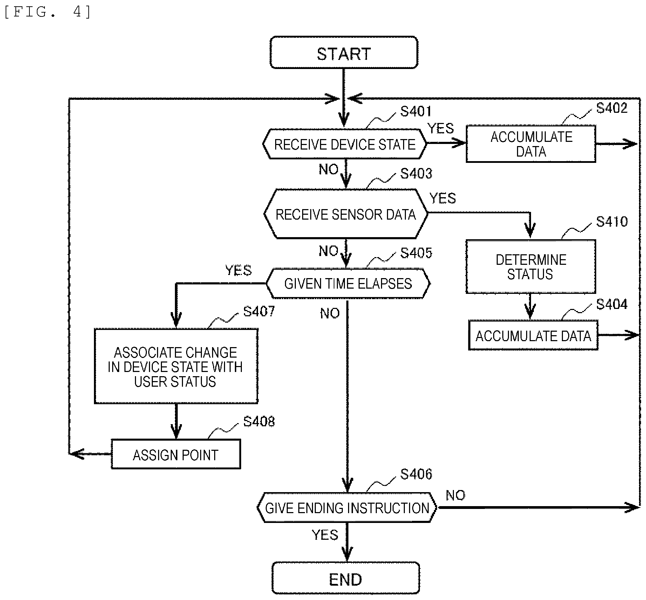

Next, a processing example of the operation management server 104 will be described with reference to the flowchart of Fig. 4. Fig. 1B is a functional block diagram illustrating the operation management server 104. The operation management server 104 includes a processing device (processor) and a storage device. A function of the operation management server 104 is implemented by the processing device (processor) executing a program stored in a storage device 400. As a physical configuration of the operation management server 104, a single server may be configured or a plurality of servers to which any part of the processing device and the storage device is connected via a network may be configured. The same function as all or some of functions configured with software may be implemented with hardware such as a field programable gate array (FPGA) or an application specific integrated circuit (ASIC).

Fig. 1B is the functional block diagram mainly illustrating functional characteristics implemented by the operation management server 104. Functional blocks include a data accumulation unit 301, a notifying unit 302, a status estimation unit 303, and a handler identifying unit 304. The data accumulation unit 301 accumulates data from the automated analysis device 101 or the mobile device 102 in the storage device 400. The notifying unit 302 controls display of the mobile device 102, as described in Fig. 6, to request the handling and the help from the laboratory technician. It is preferable to match a timing at which the notifying unit 302 controls display of the mobile device 102 with updating of display of the device state and handler list display terminal 103, a timing at which the device state 502 is updated (S306) with the handling request, and a timing at which the emphasis display (S304) is performed when the planned handler is registered and the device state still represents that an alarm is being generated and continues for a predetermined period or more with the help request. The status estimation unit 303 estimates the status of the laboratory technician holding the mobile device based on the sensor data from the mobile device 102. The estimated status includes the location and the action of the laboratory technician. The handler identifying unit 304 identifies the laboratory technician actually performing the work for resolving the alarm of the automated analysis device.

The storage device 400 is a nonvolatile memory such as a hard disk drive (HDD) or a solid state drive (SSD) and stores data necessary for a process of a system and data obtained as a result of the process. The details of the data will be described below.

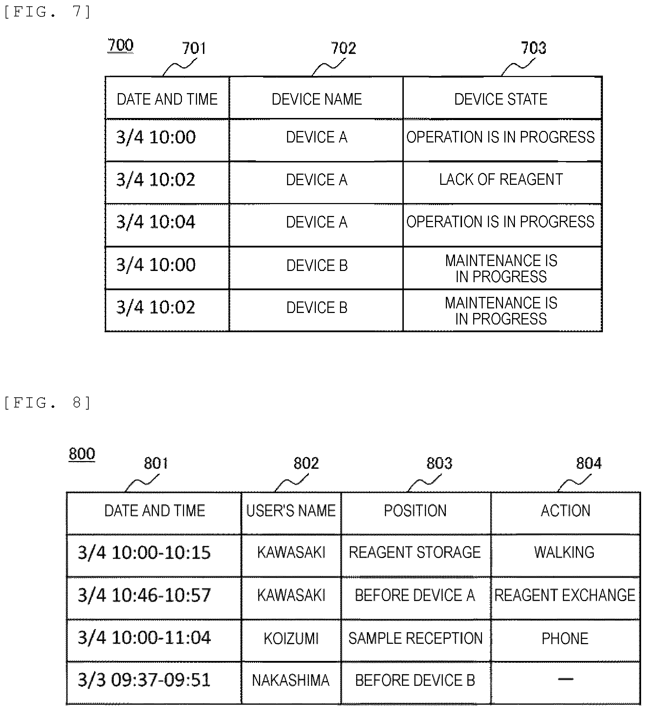

When the device state data 201 is received from the automated analysis device 101 (S401), the data accumulation unit 301 accumulates the device state data in a device state management table 700 (step 402). Fig. 7 illustrates a configuration example of the device state management table 700. The device state management table 700 is a list that includes a date and time 701, a device name 702, and a device state 703. In the device state 703, information indicating that an operation is in progress, stopping is in progress, maintenance is in progress, calibration is in progress, or an alarm is being generated, which are acquired from the device state data 201 is stored. When an alarm is being generated, the alarm content ("lack of reagent" or the like) is inclusively stored.

When the sensor data 203 and sensor data 205 from the mobile device 102 are received (S403), the data accumulation unit 301 accumulates the sensor data 203 and the sensor data 205 in a sensor data storage unit 410, and the status estimation unit 303 determines the status of the laboratory technician based on the sensor data 203 and the sensor data 205 (S410) and accumulates the data in a user status management table 800 (S404).

Fig. 8 illustrates a configuration example of the user status management table 800. The user status management table 800 is a list that includes a date and time 801, a user's name 802, a user position 803, and a user action 804. In the user position 803, a location of a user determined using the sensor data is stored. In the user action 804, an action of the user determined using the sensor data is stored.

Subsequently, it is inspected whether the given time elapses from previous check (for example, every 5 seconds) (S405). When the given time does not elapse, the following processing is performed. When the system manager inputs the ending instruction command (S406), the processing program ends. When the given time elapses from the previous check in step S405, the handler identifying unit 304 associates a change in the device state with a user status (S407). That is, the data of the device state management table 700 and the user status management table 800 is used to inspect whether there is a device in which a device state is changed from a state where an alarm is being generated to a state where an operation is in progress and there is newly a user performing a work corresponding to the resolving of the alarm of the device immediately before a period of time of the change. For example, when there is the data in which the state of the automated analysis device A is changed from a state where an alarm is being generated at 11:00 (lack of reagent) to a state where an operation is in progress, it is inspected whether there is a record in which the user position 803 approaches "device A" and the user action 804 is reagent exchange for a period of time from 10:50 to 11:00. When there is the record, the user's name 802 is extracted. When there is such a user, it is inspected whether the user is the planned handler. When the user is different from the planned handler, a point is assigned to the user and is recorded in the point management table 900 (S408) .

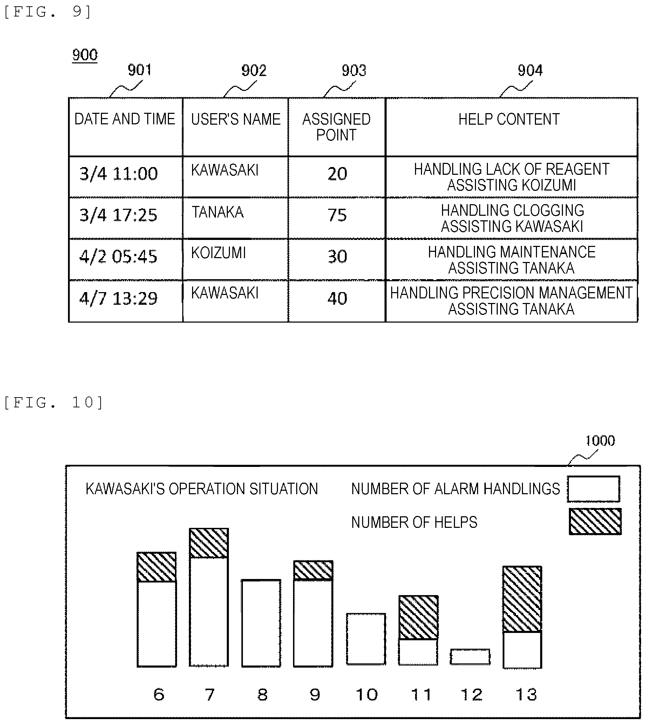

Fig. 9 illustrates a configuration example of the point management table 900. The point management table 900 is a list of rows including a date and time 901, a user's name 902, an assigned point 903, and a help content 904 performed when the point is assigned. In the help content 904, information indicating which work is performed and who is helped, as a work for handling which device and which device state, is stored.

As a method of assigning a point, as the difficulty in resolving an alarm is higher, the point may be set to be larger. Also, as a time from generation to resolution of an alarm is shorter, the point may be set to be larger. Also, depending on a period of time of work, for example, if the period of time is in the morning when the laboratory technician is busy with the operation, the point may be set to be larger. When it is determined that a plurality of laboratory technicians perform work, points may be distributed to the corresponding plurality of people.

Fig. 10 illustrates a screen example of the operation analysis terminal 105. On a screen 1000, a bar graph in which the number of alarm handlings and the number of helps are piled up is shown for each period of time of a certain laboratory technician (for example, Mr. or Ms. Kawasaki). The operation analysis terminal 105 can display a table or a graph of various analysis results by acquiring the point management table 900 in the operation management server 104 and collecting and processing the point management table 900.

In the foregoing embodiment, the example in which the work completion is determined using the sensor data is described, but the invention is not limited to the illustrated sensor. Data capable of objectively indicating work completion rather than just self-reporting may be used widely as sensor data. For example, a worker captures a photo of a device after work completion using the mobile device 102, and transmits the photo to a server. The server side may determine that an alarm state is resolved by analyzing an image of the photo.

In the foregoing present embodiment, the example in which a button displayed on a smartphone or the like is pressed, as a method of registering the planned handler or the helper, is described, but the invention is not limited thereto. The planned handler or the helper may be registered according to another method. For example, when "I will perform handling" is spoken by a voice, the content may be determined through voice recognition and may be registered as the planned handler. A keyword such as "Assist" may be extracted from a text chat and may be registered as the helper. The invention is not limited to self-registration and another person (for example, a supervisor of the laboratory technician) may perform registration.

According to the foregoing embodiment, since a state for each automated analysis device, the planned handler, and the status of the planned handler are displayed as a list in the device state and handler list display terminal 103, the laboratory technician knows at a glance which device is in an abnormal state and whether the planned handler is likely to be able to perform handling. When the planned handler is unlikely to be able to perform handling, the laboratory technician who can afford to perform assistance can perform handling instead, which promotes cooperation between laboratory technicians. Further, the change in the device state is associated with the status of the laboratory technician and it can be estimated who actually performs work to resolve an abnormal state of the device from objective data. Therefore, it is possible to correctly assess the laboratory technician who actually performs the work for resolving an alarm.

### Reference Signs List

- 101:: automated analysis device
- 102:: mobile device
- 103:: device state and handler list display terminal
- 104:: operation management server
- 105:: operation analysis terminal
- 106:: wired network
- 107:: wireless access point
- 201:: device state data
- 203, 205:: sensor data
- 301:: data accumulation unit
- 302:: notifying unit
- 303:: status estimation unit
- 304:: handler identifying unit
- 400:: storage device
- 410:: sensor data storage unit
- 500, 600, 1000:: screen
- 501:: device name
- 502:: device state
- 503:: planned handler
- 504:: status of planned handler
- 505:: elapsed time
- 506:: helper
- 601, 602:: list
- 603:: help button
- 604:: handling button
- 700:: device state management table
- 701:: date and time
- 702:: device name
- 703:: device state
- 800:: user status management table
- 801:: date and time
- 802:: user's name
- 803:: position
- 804:: action
- 900:: point management table
- 901:: date and time
- 902:: user's name
- 903:: assigned point
- 904:: help content

## Claims

1. A laboratory operation management system comprising:
a plurality of automated analysis devices that are installed in a laboratory and configured to analyze biological samples;
a mobile device that is held by each laboratory technician performing an operation in the laboratory; and
an operation management server that includes a data accumulation unit, a notifying unit, a status estimation unit, and a handler identifying unit, wherein
the data accumulation unit accumulates device state data indicating a device state regularly transmitted from each of the plurality of automated analysis devices in a storage device,
the notifying unit requests handling from the mobile device when the device state indicated by the device state data transmitted from a first automated analysis device represents that an alarm is being generated,
the status estimation unit estimates a status including a location and an action of a first laboratory technician holding a first mobile device on the basis of sensor data regularly transmitted from the first mobile device that registers a planned handler in response to the request for the handling from the notifying unit, and accumulates the status in the storage device,
the notifying unit requests help from the mobile device when the device state indicated by the device state data transmitted from the first automated analysis device still represents that the alarm is being generated and continues for a predetermined period or more even after the planned handler is registered,
the status estimation unit estimates a status including a location and an action of a second laboratory technician holding a second mobile device on the basis of sensor data regularly transmitted from the second mobile device that registers a helper in response to the help from the notifying unit, and accumulates the status in the storage device, and
the handler identifying unit identifies a handler for resolving the alarm of the first automated analysis device on the basis of the status of the first laboratory technician and the status of the second laboratory technician immediately before a timing at which the alarm of the first automated analysis device accumulated in the storage device is resolved.

2. The laboratory operation management system according to claim 1, wherein the handler identifying unit assigns a point to the second laboratory technician when the handler for resolving the alarm of the first automated analysis device is identified as the second laboratory technician.

3. The laboratory operation management system according to claim 1, wherein the handler identifying unit identifies a work content for resolving the alarm of the first automated analysis device and the handler for resolving the alarm of the first automated analysis device by comparing the status of the first laboratory technician with the status of the second laboratory technician immediately before the timing at which the alarm of the first automated analysis device is resolved.

4. The laboratory operation management system according to claim 1, further comprising:
a display terminal, wherein
the display terminal displays a device state indicated by latest device state data from the plurality of automated analysis devices, displays an elapsed time from first reception of the device state data representing that the alarm is being generated when the device state indicated by the device state data represents that the alarm is being generated, and emphasizes and displays information regarding the automated analysis device when the elapsed time satisfies a predetermined condition.

5. The laboratory operation management system according to claim 4, wherein
when the planned handler is registered, the display terminal displays a laboratory technician who registers the planned handler and a status of the laboratory technician as information regarding the automated analysis device, and
when the helper is registered, the display terminal displays a laboratory technician who registers the helper as information regarding the automated analysis device.

6. The laboratory operation management system according to claim 5, wherein the display terminal emphasizes and displays the information regarding the automated analysis device when a given time or more elapses after the planned handler is registered and the device state of the automated analysis device still represents that the alarm is being generated and continues for a predetermined period or more or when the device state data representing that the alarm is being generated is first received and then the predetermined period elapses and the planned handler is not registered.

7. An operation management server that manages an operation performed by a laboratory technician in a laboratory where a plurality of automated analysis devices analyzing biological samples are installed, the operation management server comprising:
a data accumulation unit;
a notifying unit;
a status estimation unit; and
a handler identifying unit, wherein
the data accumulation unit accumulates device state data indicating a device state regularly transmitted from each of the plurality of automated analysis devices in a storage device,
the notifying unit requests handling from a mobile device held by each laboratory technician when the device state indicated by the device state data transmitted from a first automated analysis device represents that an alarm is being generated,
the status estimation unit estimates a status including a location and an action of a first laboratory technician holding a first mobile device on the basis of sensor data regularly transmitted from the first mobile device that registers a planned handler in response to the request for the handling from the notifying unit, and accumulates the status in the storage device,
the notifying unit requests help from the mobile device when the device state indicated by the device state data transmitted from the first automated analysis device still represents that the alarm is being generated and continues for a predetermined period or more even after the planned handler is registered,
the status estimation unit estimates a status including a location and an action of a second laboratory technician holding a second mobile device on the basis of sensor data regularly transmitted from the second mobile device that registers a helper in response to the help from the notifying unit, and accumulates the status in the storage device, and
the handler identifying unit identifies a handler for resolving the alarm of the first automated analysis device on the basis of the status of the first laboratory technician and the status of the second laboratory technician immediately before a timing at which the alarm of the first automated analysis device accumulated in the storage device is resolved.

8. The operation management server according to claim 7, wherein the handler identifying unit assigns a point to the second laboratory technician when the handler for resolving the alarm of the first automated analysis device is identified as the second laboratory technician.

9. The operation management server according to claim 7, wherein the handler identifying unit identifies a work content for resolving the alarm of the first automated analysis device and the handler for resolving the alarm of the first automated analysis device by comparing the status of the first laboratory technician with the status of the second laboratory technician immediately before the timing at which the alarm of the first automated analysis device is resolved.

10. An operation management method in which an operation performed by a laboratory technician in a laboratory where a plurality of automated analysis devices analyzing biological samples are installed is managed using an operation management server including a data accumulation unit, a notifying unit, a status estimation unit, and a handler identifying unit, the operation management method comprising:
by the data accumulation unit,
accumulating device state data indicating a device state regularly transmitted from each of the plurality of automated analysis devices in a storage device;
by the notifying unit,
requesting handling from a mobile device held by each laboratory technician when the device state indicated by the device state data transmitted from a first automated analysis device represents that an alarm is being generated;
by the status estimation unit,
estimating a status including a location and an action of a first laboratory technician holding a first mobile device on the basis of sensor data regularly transmitted from the first mobile device that registers a planned handler in response to the request for the handling from the notifying unit, and accumulates the status in the storage device;
by the notifying unit,
requesting help from the mobile device when the device state indicated by the device state data transmitted from the first automated analysis device still represents that the alarm is being generated and continues for a predetermined period or more even after the planned handler is registered;
by the status estimation unit,
estimating a status including a location and an action of a second laboratory technician holding a second mobile device on the basis of sensor data regularly transmitted from the second mobile device that registers a helper in response to the help from the notifying unit, and accumulates the status in the storage device; and
by the handler identifying unit,
identifying a handler for resolving the alarm of the first automated analysis device on the basis of the status of the first laboratory technician and the status of the second laboratory technician immediately before a timing at which the alarm of the first automated analysis device accumulated in the storage device is resolved.

11. The operation management method according to claim 10, further comprising:
by the handler identifying unit,
assigning a point to the second laboratory technician when the handler for resolving the alarm of the first automated analysis device is identified as the second laboratory technician.

12. The operation management method according to claim 10, further comprising:
by the handler identifying unit,
identifying a work content for resolving the alarm of the first automated analysis device and the handler for resolving the alarm of the first automated analysis device by comparing the status of the first laboratory technician with the status of the second laboratory technician immediately before the timing at which the alarm of the first automated analysis device is resolved.
